# EUROPEAN PATENT APPLICATION

(11) **EP 3 219 359 A1**
(43) Date of publication of application: **20.09.2017**
(21) Application number: 17160853.2
(22) Date of filing: 14.03.2017
(51) Int. Cl.: A61N 1/39, A61B 5/021, A61B 5/024, A61B 5/1455

(54) **AUTOMATIC EXTERNAL DEFIBRILLATOR**

(30) Priority: 14.03.2016 JP 2016049591
(71) Applicant: Fujita Medical Instruments Co., Ltd., Tokyo 113-0033 (JP)
(72) Inventor: MAEDA, Hironobu, TOKYO, 113-0033 (JP); YAMAMURA, Haruo, TOKYO, 113-0033 (JP)
(74) Representative: Epping - Hermann - Fischer

(57) **Abstract**

There is provided an automatic external defibrillator including: an AED body that has a pair of defibrillation pads pasted on a chest of a rescuee so as to give an electric shock to the rescuee; and a pulse wave sensor unit that is disposed in the AED body so as to detect a pulse wave in a head of the rescuee.

## Description

### BACKGROUND

### FIELD OF THE INVENTION

The present invention relates to an automatic external defibrillator, and particularly relates to an automatic external defibrillator which detects whether proper chest compression is performed.

### BACKGROUND ART

Conventionally, an automatic external defibrillator (hereinafter, referred to as an "AED") has been known in which a cardiogram is measured by pasting a pad to a chest so as to automatically perform defibrillation, based on a waveform analysis. As a preliminary step when this AED is utilized, cardiopulmonary resuscitation (chest compression and artificial respiration) is performed artificially. In the cardiopulmonary resuscitation, when the chest compression which is so-called cardiac massage is performed, a depth and a tempo of the chest compression are important factors. In a case of adults, it is normally recommended that the depth of the chest compression is 2 inches (5 cm) or longer. The chest compression has to be performed under a condition that the tempo of compression times is 100 to 120 times per minute (refer to Guideline for Cardiopulmonary Resuscitation and Emergency Cardiovascular Treatment, Revised Edition in 2015).

On the other hand, in a case of the recent AED, a technique has been known in which whether the above-described cardiopulmonary resuscitation is performed artificially by a proper method is fed back using an acceleration sensor (for example, refer to Japanese Patent Application Laid-Open Publication No. 2005-046609). This AED has a function to measure speed and a depth degree of the chest compression, and to give an audio advice to a practitioner with regard to whether the chest compression is properly performed.

### SUMMARY

Incidentally, according to a method of determining proper timing for a defibrillation device to apply a shock voltage, a problem is pointed out in that it is difficult to achieve return of spontaneous circulation (ROSC) if intracerebral oxygen saturation does not reach 40% to 50% or greater. Therefore, it is also suggested that a prognosis of a resuscitated patient is improved if the chest compression is continuously performed until the intracerebral oxygen saturation reaches 40% to 50% or greater.

However, according to the AED using the above-described acceleration sensor, even if the depth and the tempo of the chest compression are ideal in a clinical site, it is not possible to actually recognize whether an advantageous effect of the AED is obtained. That is, although the acceleration sensor may recognize the depth and the tempo, in fact, it is not possible to obtain information indicating evidence that blood is really circulated by the chest compression.

The present invention is made in order to solve the above-described problem, and an object thereof is to provide an automatic external defibrillator which may recognize whether or not blood is really circulated by chest compression, through a measurement analysis in which blood pulsating information is acquired as a pulse wave by using a sensor.

According to the present invention, there is provided an automatic external defibrillator including an AED body that has a pair of defibrillation pads pasted on a chest of a rescuee so as to give an electric shock to the rescuee, and a pulse wave sensor unit that is disposed in the AED body so as to detect a pulse wave in a head of the rescuee.

According to the present invention, it is possible to recognize whether or not the blood is really circulated by the chest compression, through the measurement analysis in which the blood pulsating information is acquired as the pulse wave by using the sensor.

### BRIEF DESCRIPTION OF THE DRAWINGS

Fig. 1 is a schematic diagram illustrating a usage state of an automatic external defibrillator according to an exemplary embodiment of the present invention.
Fig. 2 is a block diagram of another automatic external defibrillator according to the exemplary embodiment of the present invention.

### DETAILED DESCRIPTION

Hereinafter, an exemplary embodiment according to the present invention will be described with reference to the drawings.

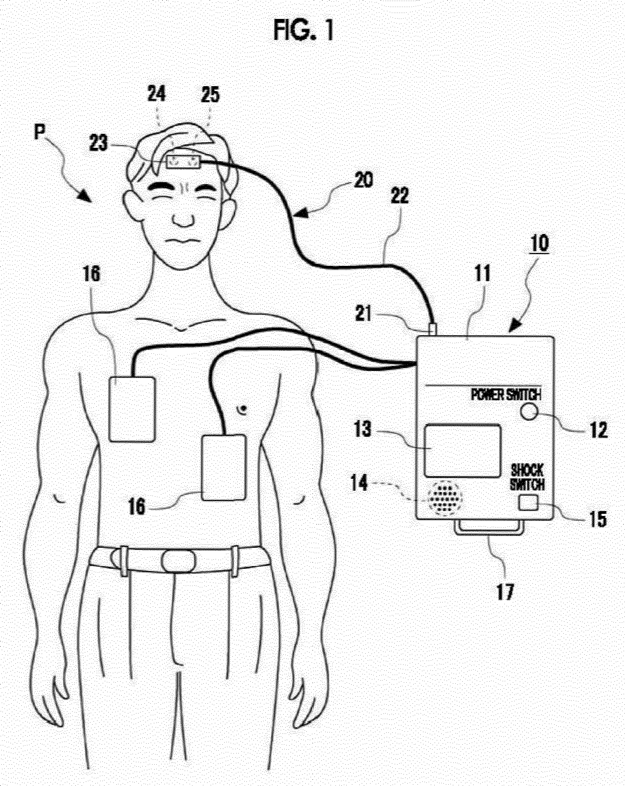

As illustrated in Fig. 1, an automatic external defibrillator (hereinafter, referred to as an "AED") 10 includes an AED body 11 stored in a storage case (not illustrated), a power switch (SW) 12 disposed in the AED body 11, a display unit 13, an audio unit 14, a shock switch (SW) 15, a pair of defibrillation pads 16, and a pulse wave sensor unit 20.

The AED body 11 is a molded product formed of a hard resin, and internally stores a control unit (to be described later). The AED body 11 integrally includes a handgrip 17 so as to be portable.

The power switch 12 has a function as a main switch to drive the control unit of the AED body 11. For example, the power switch 12 is configured to be automatically turned on when the AED body 11 is taken out from the storage case (not illustrated). In some cases, a manually operated switch may not be provided.

For example, the display unit 13 outputs a confirmed result of a state of a rescuee P or display guidance such as an operation procedure, to a rescuer (not illustrated).

The audio unit 14 is disposed inside the AED body 11, and outputs the confirmed result of the state of the rescuee P or audio guidance such as the operation procedure and attention, to the rescuer (not illustrated).

The shock switch 15 has a function as a trigger switch for giving the electric shock in a case where the control unit determines that the state of the rescuee P needs the shock. The shock switch 15 has an electrically locking function so that the rescuer does not activate the AED by mistake.

The pair of defibrillation pads 16 is pasted on the chest (human head) of the rescuee P, thereby acquiring biological information of the rescuee P such as a cardiac potential and giving the electric shock to the rescuee P.

The power switch 12, the display unit 13, the audio unit 14, the shock switch 15, and the pair of defibrillation pads 16 have the same function as those which are mounted on a known AED 10, and may be optionally arranged.

The pulse wave sensor unit 20 includes a connector 21 attached to the AED body 11 so as to be detachable therefrom, a wire 22 whose one end is connected to the connector 21, a pulse wave sensor pad 23 disposed in the other end of the wire 22, and a pair of light emitting unit 24 and light receiving unit 25 disposed in the pulse wave sensor pad 23. For example, as the connector 21, a USB connector or an RS232C interface connector may be used. If the USB connector or the RS232C interface connector which is provided with a medical standard insulation structure is used for the connection to the AED body 11, the connector may be easily connected to the AED body 11, and furthermore, the connector may function as an embedded device. That is, in order for the pulse wave sensor unit 20 to be applicable to the existing AED 10, it is necessary to employ a module which may sufficiently withstand a shock voltage generated by the AED 10. Therefore, the module may be provided with a CF-type mounting unit conforming to a safety plan of a medical device.

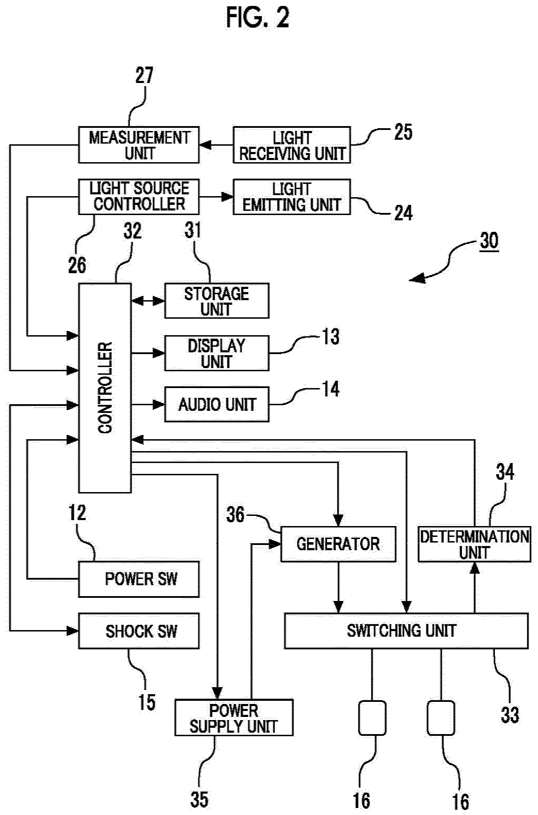

Here, a configuration of the above-described control unit will be described with reference to Fig. 2. In Fig. 2, a control unit 30 according to the exemplary embodiment has a computer configuration including a storage unit 31 which stores a program according to the exemplary embodiment, and a controller 32 which executes various processes in accordance with the program stored in the storage unit 31.

For example, if the power switch 12 is turned on, the controller 32 controls the display unit 13 and the audio unit 14 so as to execute the guidance in accordance with the program stored in the storage unit 31.

The controller 32 causes at least any one of the display unit 13 and the audio unit 14 to send each guidance such as an instruction that the pulse wave sensor unit 20 is to be mounted on the AED body 11 and an instruction that the pulse wave sensor pad 23 is to be pasted on a forehead (head) of the rescuee P. Furthermore, the controller 32 controls a light source controller 26 so as to emit near-infrared light from the light emitting unit 24. The controller 32 acquires biological information relating to a pulse wave measured by a measurement unit 27 based on reflected light received by the light receiving unit 25. In this manner, controller 32 controls at least any one of the display unit 13 and the audio unit 14 in accordance with the biological information relating to the pulse wave output from the pulse wave sensor unit 20.

On the other hand, the controller 32 controls a switching unit 33, and acquires a determination result relating to whether the shock is required based on the biological information of the rescuee P such as the cardiac potential output from the defibrillation pad 16 pasted on the chest of the rescuee P, from a determination unit 34.

Here, in a case where the controller 32 acquires the determination result that the shock is required from the determination unit 34, the controller 32 controls a power supply unit 35 so as to charge a generator 36 with power supplied from a battery (not illustrated). If the charging is completed, the controller 32 permits the shock switch 15 to receive an operation, and controls the switching unit 33 so as to switch a state of the defibrillation pad 16 from an acquired state of the biological information to a shock state where the electric shock is given to the rescuee P. Then, the rescuer operates the shock switch 15 so that the controller 32 gives the electric shock to the rescuee P by using the power which has been charged to the generator 36.

A light emitting diode (LED) is used for the light emitting unit 24, and a photodiode (PD) is used for the light receiving unit 25. The light emitting unit 24 emits light during measurement in accordance with a light emitting signal output from the light source controller 26, and emits near-infrared light (for example, pulsed light) to the inside a living body (into a brain). The light receiving unit 25 receives light reflected from the inside of the living body at timing synchronized with the near-infrared light emitted toward the inside of the living body. For example, a light emitting element of the light emitting unit 24 may not be the LED, and may be a laser diode as long as the light emitting element may sequentially output light having a plurality of wavelengths in a near-infrared region.

The light source controller 26 outputs an instruction signal for instructing to drive the light emitting unit 24, to the light emitting unit 24. The instruction signal includes information such as intensity or a wavelength (for example, any one wavelength of 770 nm, 800 nm, and 870 nm) of the near-infrared light emitted by the light emitting unit 24. Based on a drive signal received from the controller 32, the light source controller 26 controls the light emission of the light emitting unit 24.

In the exemplary embodiment, the measurement unit 27 measures oxygen saturation rSO2 and a hemoglobin index HbI of intracerebral blood. The hemoglobin index HbI reflects a change in the amount of hemoglobin of a capillary bed. If the rescuee P is subjected to the chest compression by a rescuer, a heart is pressed from above a sternum, and blood present in the heart is pressed out. If the chest compression is released, a valve inside the heart is closed, the blood does not reversely flows, and new blood is fed to the heart through a pulmonary artery. Then, the sternum is repeatedly compressed and decompressed, thereby allowing the blood to be circulated in the brain or organs. Therefore, it is possible to contribute to the resuscitation of the rescuee P.

Thus, the measurement unit 27 performs the measurement analysis on blood pulsating information obtained during the measurement of the hemoglobin index HbI, and acquires the information as a pulse wave. In this manner, the measurement unit 27 may identify a peak or a valley of a waveform in the pulse wave.

At peak timing or valley timing of the waveform in the pulse wave, the display unit 13 or the audio unit 14 outputs the guidance, thereby enabling a rescuer to recognize a chest compression tempo thereof and a fact that the blood apparently flows therein.

In this case, the chest compression tempo serves as evidence indicating a result obtained from the blood which is really fed from the heart into the brain. Accordingly, unlike the result simply obtained from the tempo of the moving sternum by the acceleration sensor, it is possible to improve reliability.

In this way, the pulse wave sensor unit 20 is used, and thus, it is possible to match a cycle of a rescuer performing the chest compression so that the tempo based on the information relating to the pulse wave obtained from the hemoglobin index HbI reaches 100 to 120 times per minute. At the same time, it is possible to confirm that the pulse wave is output, that is, that the heart is accurately pressed. Therefore, it is possible to effectively perform the chest compression.

Furthermore, during the chest compression, the oxygen saturation rSO2 of the intracerebral blood is measured. Accordingly, it is possible to prompt an operation for intensively and repeatedly performing the chest compression until the oxygen saturation rSO2 reaches a value which enables return of spontaneous circulation (ROSC) through cardiopulmonary resuscitation. In this manner, it is possible to contribute to an omitted operation of a rescuer to touch a carotid artery or an omitted operation of the rescuer to check respiration. Therefore, it is possible to easily bring the rescuee P into a state enabling the ROSC at an early stage.

In this basic configuration, the automatic external defibrillator 10 according to the exemplary embodiment includes the AED body 11 that has the pair of defibrillation pads 16 which is pasted on the chest of the rescuee P so as to give the electric shock, and the pulse wave sensor unit 20 that is disposed in the AED body 11 so as to detect the pulse wave in the head of the rescuee P. In this manner, the sensor performs the measurement analysis on the blood pulsating information, and acquires the information as the pulse wave, thereby recognizing whether or not the blood is really circulated by the chest compression.

Next, a usage example of using the AED 10 according to the exemplary embodiment will be described. In the following procedures to be performed in the alphabetical order, the resuscitation is performed using the AED 10 in a case of having no mounted pulse wave sensor unit 20 according to the exemplary embodiment.
(A) Check the reaction of the rescuee P
(B) Call for help
(C) Secure the airway of the rescuee P
(D) Check the respiration of the rescuee P
(E) Perform artificial respiration on the rescuee P
(F) Give cardiac massage to the rescuee P
(G) Turn on the power switch 12 of the AED 10
(H) Paste the defibrillation pad 16 on the chest of the rescuee P
(I) Analyze the cardiogram of the rescuee P
(J) Give the electric shock to the rescuee P

In contrast, in the following procedures to be performed in the numerical order, the resuscitation is performed using the AED 10 in a case of having the mounted pulse wave sensor unit 20. The following procedures are prepared only for a case where a rescuer performs life-saving treatment by using the AED 10. When the life-saving treatment is performed by a doctor or an appropriate person who can properly perform the chest compression, even if the same AED 10 is used, the pulse wave sensor unit 20 may not be used.
(1) Check the reaction of the rescuee P
(2) Call for help
(3) Secure the airway of the rescuee P
(4) Check the respiration of the rescuee P
(5) Paste the pulse wave sensor pad 23 on the head of the rescuee P
(6) Turn on the power switch 12 of the AED 10
(7) Perform artificial respiration on the rescuee P
(8) Give cardiac massage to the rescuee P
(9) Paste the defibrillation pad 16 on the chest of the rescuee P
(10) Analyze the cardiogram of the rescuee P
(11) Give the electric shock to the rescuee P

For example, the procedures (5) to (7) in the above-described order may not be performed in this order. If the above-described procedure (5) is used, a life-saving operation performed by a rescuer is the same as the known life-saving operation except that the procedure (6) of turning on the power switch is performed prior to the procedure (8) of giving the cardiac massage. Therefore, in the following description, a case will be specifically described where the cardiac massage is given to the rescuee P in the procedure (8).

If the pulse wave sensor pad 23 of the pulse wave sensor unit 20 serving as a near-infrared oxygen saturation monitor is pasted on the head of the rescuee P, near-infrared light using near-infrared rays having three different wavelengths is emitted 10 times per second from the light emitting unit 24.

The near-infrared light is reflected on the inside of the brain at a depth degree of 70% to 80% (for example, 30 to 40 mm) of a separated distance between the light emitting unit 24 and the light receiving unit 25. The reflected light is received by the light receiving unit 25.

Based on the reflected light received by the light receiving unit 25, information of an absorption change caused by hemoglobin is measured in a time division manner by the measurement unit 27.

For example, in order to obtain information of the intracerebral blood, an increase or a decrease in the near-infrared light of 800 nm may be directly measured. However, if an emission interval of the near-infrared light is set to approximately 10 times per second, it is difficult to clearly distinguish the peak and the valley of the waveform. Therefore, in the exemplary embodiment, three different wavelengths including the 800 nm are used. If all of the waveforms rise or fall, the measurement unit 27 may determine whether all of the waveforms rise or fall as the pulse wave.

Therefore, if light receiving results obtained by the measurement unit 27 for all of the employed three wavelengths using the near-infrared light are summed, the sum is 30 times per second. Accordingly, it is possible to sufficiently detect the peak or the valley of the waveform whose cycle per second is 120 times, that is, twice per second.

In this manner, the controller 32 uses the peak or the valley of the waveform so as to detect whether or not the blood is fed into the brain by the chest compression, and uses the display unit 13 and the audio unit 14 to notify a rescuer of a chest compression rhythm. In this manner, the controller 32 may recognize whether the chest compression is properly performed.

At this time, for example, even in a case where there is a possibility that the chest compression may not be sufficiently performed merely by using a compression sensor, such as a case where strength of the chest compression is insufficient for a fat person, and a case where a position of the chest compression is misaligned, it is possible to recognize whether the chest compression is properly performed.

Then, the controller 32 controls the light source controller 26 and the measurement unit 27 so that the light emitting unit 24 and the light receiving unit 25 are synchronized with each other. The measurement unit 27 collects and records a signal having a plurality of wavelengths received by the light receiving unit 25 in a time division manner, and obtains a ratio R/IR of an absorbance degree of each wavelength. The measurement unit 27 calculates the oxygen saturation of the blood and a relative value of the hemoglobin amount, stores both of them in the storage unit 31, and causes the display unit 13 to display both of them. As a measurement principle, the calculation is performed based on the Beer Lambert's law in which the absorbance degree of the near-infrared ray inside the living body is proportional to concentration of the hemoglobin contained in the tissue.

Therefore, when a determination result is output which shows that the blood sufficiently flows into the brain based on the determination result of the measurement unit 27, or when the controller 32 determines that the blood sufficiently flows into the brain based on the measurement result of the measurement unit 27, the controller 32 starts charging, and causes the display unit 13 and the audio unit 14 to notify a rescuer to give the shock to the rescuee P. Further, for example, the controller 32 notifies the rescuer of a state where the shock switch 15 is operable or notifies the rescuer to take steps away from the rescuee P. For example, when the measurement unit 27 determines that the measurement value for the oxygen saturation of the rescuee P exceeds a predetermined reference value (for example, 40%), the controller 32 uses the display unit 13 and the audio unit 14 as a notification unit so as to notify the rescuer to give the shock to the rescuee P in response to the determination.

In this manner, it is possible for the rescuer to give the shock to the rescuee P by operating the shock switch 15.

In this way, the automatic external defibrillator 10 according to the exemplary embodiment includes the AED body 11 that has the pair of defibrillation pads 16 which is pasted on the chest of the rescuee P so as to give the electric shock, and the pulse wave sensor unit 20 that is disposed in the AED body 11 so as to detect the pulse wave in the head of the rescuee P. In this manner, the sensor performs the measurement analysis on the blood pulsating information, and acquires the information as the pulse wave. Accordingly, it is possible to recognize whether or not the blood is really circulated by the chest compression.

Incidentally, the automatic external defibrillator 10 according to the present invention is not limited to the above-described exemplary embodiment, and includes various design modifications within the technical scope described in claims, and within the scope not departing from the gist of the invention.

For example, with regard to the separated distance between the light emitting unit 24 and the light receiving unit 25, it is possible to change a depth of the living body tissue which is measurable by the separated distance. It is possible to measure the depth of 70% to 80% of a distance between centers of the light emitting unit 24 and the light receiving unit 25. Therefore, for example, a plurality of the sensor units having different separated distances may be prepared for adults and children. As necessary, the sensor unit may be attached to the AED body 11 so as to be detachable therefrom.

An example has been described in which the automatic external defibrillator 10 uses the pair of light emitting units 24 and light receiving units 25 to measure the biological information of the head of the rescuee P. However, it is possible to check information of the right and left brains if each pair is disposed on the right and left. In this case, for example, it is possible to change a depth degree for the inside of the living body by using a measurement position of the biological information obtained by one pair of light emitting unit and light receiving unit and a measurement position of the biological information obtained by the other pair of light emitting unit and light receiving unit.

As described above, the automatic external defibrillator according to the present invention has an advantageous effect in that it is possible to recognize whether or not the blood is really circulated by the chest compression by causing the sensor to perform the measurement analysis on the blood pulsating information and to acquire the information as the pulse wave. The present invention is usefully applicable to all of the automatic external defibrillators which detect whether the chest compression is properly performed. Through the monitoring of the oxygen saturation, it is known that, in a case of a reference value (for example, 50% or smaller), the return of spontaneous circulation (ROSC) is not often achieved by the cardiopulmonary resuscitation. Accordingly, in a case of the reference value or smaller, a less effective shock is not given to the rescuee P. In this manner, it is possible to expect an advantageous effect in that a prognosis of the resuscitated rescuee P does not become worse. In a case where the ROSC is not achieved even if the shock is given to the rescuee P, myocardium is exhausted, thereby causing a risky situation where the prognosis becomes worse.

## Claims

1. An automatic external defibrillator comprising:
an AED body that has a pair of defibrillation pads pasted on a chest of a rescuee so as to give an electric shock to the rescuee; and
a pulse wave sensor unit that is disposed in the AED body so as to detect a pulse wave in a head of the rescuee.

2. The automatic external defibrillator according to claim 1,
wherein the pulse wave sensor unit comprises a light emitting unit that emits near-infrared light in a near-infrared region toward a human head, and a light receiving unit that comes into contact with the human head so as to receive reflected light of the near-infrared light which has been emitted from the light emitting unit and reflected from the human head.

3. The automatic external defibrillator according to any one of claims 1 and 2,
wherein the pulse wave sensor unit comprises a measurement unit that measures oxygen saturation of intracerebral blood during chest compression of the rescuee.

4. The automatic external defibrillator according to any one of claims 1 to 3,
wherein the pulse wave sensor unit comprises a measurement unit that performs a measurement analysis on a blood pulsation obtained by measuring a hemoglobin index of intracerebral blood during chest compression of the rescuee.

5. The automatic external defibrillator according to claim 4,
wherein the AED body comprises a notification unit that notifies a rescuer of a chest compression operation at peak timing or valley timing of a signal waveform, based on the signal waveform obtained by the measurement unit performing the measurement analysis on the pulsation.

6. The automatic external defibrillator according to any one of claims 1 and 2,
wherein the pulse wave sensor unit comprises a measurement unit that performs a measurement analysis on a blood pulsation obtained by measuring oxygen saturation and a hemoglobin index of intracerebral blood during chest compression of the rescuee, and a notification unit that notifies the electric shock is permitted to be given to the chest of the rescuee from the pair of defibrillation pads in response to a fact that a measurement value of the oxygen saturation exceeds a predetermined reference value.
